Europäisches Patentamt

European Patent Office

Office européen des brevets

(19)

(11) Veröffentlichungsnummer: **0 339 445**

**A1**

(12) **EUROPÄISCHE PATENTANMELDUNG**

(21) Anmeldenummer: 89106972.6

(22) Anmeldetag: 19.04.89

(51) Int. Cl.⁴: **C08B 37/00 , C12P 19/04 ,**
**//(C12P19/04,C12R1:38)**

Der Anmelder hat eine Erklärung nach Regel 28 (4) EPÜ (Herausgabe einer Probe nur an einen Sachverständigen) eingereicht. Eingangsnummer(n) derHinterlegung(en) : DSM 4429

(30) Priorität: 27.04.88 DE 3814219

(43) Veröffentlichungstag der Anmeldung:
02.11.89 Patentblatt 89/44

(84) Benannte Vertragsstaaten:
AT BE CH DE FR GB IT LI NL

(71) Anmelder: HOECHST AKTIENGESELLSCHAFT
Postfach 80 03 20
D-6230 Frankfurt am Main 80(DE)

(72) Erfinder: Then, Johann, Dr.
Bechtenwaldstrasse 70
D-6230 Frankfurt am Main 80(DE)
Erfinder: Giani, Carlo, Dr.
Hedderichstrasse 69
D-6000 Frankfurt am Main 70(DE)
Erfinder: Wöhner, Gerhard, Dr.
Flörsheimer Strasse 27
D-6093 Flörsheim am Main(DE)
Erfinder: Wink, Joachim, Dr.
Bieberer Strasse 133
D-6050 Offenbach(DE)
Erfinder: Buchholz, Rainer, Dr.
Grosser Ring 38
D-5239 Unnau(DE)
Erfinder: Voelskow, Hartmut, Dr.
Akazienstrasse 22
D-6234 Hattersheim am Main(DE)
Erfinder: Schlingmann, Merten, Prof. Dr.
Schneidhainer Strasse 32a
D-6240 Königstein Taunus(DE)
Erfinder: Rapp, Knut, Dr.
Im Kerner 16
D-6521 Offstein(DE)
Erfinder: Vogel, Manfred, Dr.
Beim Bergtor 28
D-6718 Grünstadt(DE)

(54) Neues rhamnosehaltiges Polysaccharid, Verfahren zu seiner Herstellung und seine Verwendung.

(57) Pseudomonas paucimobilis DSM 4429 synthetisiert in aerober Kultur ein neues Polysaccharid mit den wesentlichen Bestandteilen L-Rhamnose, Glucose, Galaktose, Mannose und Glucuronsäure im Verhältnis 1:0,5 bis 1,8:0,04 bis 0,1:0,04 bis 0,16:0,2 bis 0,6. Aus diesem Polysaccharid kann Rhamnose leicht hydrolytisch abgespalten werden und als Vorstufe zur Herstellung verschiedener pharmazeutischer Erzeugnisse, Pflanzenschutzmittel oder Aromastoffe eingesetzt werden.

## Neues rhamnosehaltiges Polysaccharid, Verfahren zu seiner Herstellung und seine Verwendung

In jüngster Zeit finden die Eigenschaften von Sacchariden zunehmend Interesse. Sie werden immer häufiger als Ausgangsubstrate für die Synthese von pharmazeutischen Erzeugnissen und Pflanzenschutzmitteln verwendet. Auch L-Rhamnose oder deren Derivate finden immer breitere Anwendung auf diesen Gebieten, aber auch im Bereich der Cytologie von pflanzlichen und tierischen Zellen, der Mikrobiologie, Gentechnologie, Aromaherstellung und Immunologie.

Die Herstellung rhamnosehaltiger Exopolysaccharide ist in verschiedenen Patenten und Patentanmeldungen bereits beschrieben worden (DE 32 29 700; US 4,342,866 oder US 4,529,797; US 4,454,316). Danach kann die Herstellung von Rhamnose durchgeführt werden, indem Bakterien der Gattungen Alcaligenes, Klebsiella, Pseudomonas oder Enterobacter fermentiert werden, bis sie rhamnosehaltige Polysaccharide im Nährmedium anhäufen. Die Rhamnose kann dann nach saurer Hydrolyse aus diesen Polysacchariden isoliert werden.

Überraschend wurde nun gefunden, daß Pseudomonas paucimobilis ein neues, ebenfalls rhamnosehaltiges Polysaccharid synthetisiert, aus dem die gewünschte Rhamnose leicht isoliert werden kann.

Die Erfindung betrifft somit:

1. Ein Polysaccharid mit den wesentlichen Bestandteilen L-Rhamnose, Glucose, Galaktose, Mannose und Glucuronsäure, in dem Verhältnis 1(Rhamnose):0,5 bis 1,8(Glucose):0,04 bis 0,1(Galaktose):0,04 bis 0,16(Mannose):0,2 bis 0,6 (Glucuronsäure).

2. Ein Verfahren zur Herstellung des unter 1 charakterisierten Polysaccharids durch Kultivierung eines Pseudomonasstamms, dadurch gekennzeichnet, daß Pseudomonas paucimobilis DSM 4429 eingesetzt wird.

3. Die Verwendung des unter 1 charakterisierten Polysaccharids zur Herstellung von Rhamnose.

Im folgenden wird die Erfindung detailliert erläutert, insbesondere in ihren bevorzugten Ausführungsformen. Ferner wird die Erfindung in den Ansprüchen definiert.

Pseudomonas paucimobilis DSM 4429 wurde am 8.3.1988 bei der Deutschen Sammlung von Mikroorganismen unter der angegebenen Nummer nach den Bestimmungen des Budapester Vertrages hinterlegt. Der Stamm wurde ursprünglich aus Erdproben isoliert, wobei das Isolationskriterium die Ausbildung von Kapseln oder Schleimen auf kohlenhydratreichen Nährmedien war.

Pseudomonas paucimobilis DSM 4429 hat folgende Eigenschaften:

| Zellform | Stäbchen |
|---|---|
| Breite µm | 0.7 - 0.8 |
| Länge µm | 1.2 - 1.6 |
| Beweglichkeit | + |
| Geißeln | polar 1 |
| Gram-Reaktion | - |
| Lyse durch 3 % KOH | + |
| Aminopeptidase (Bestimmung nach Cerny) | + |
| Sporen | - |
| Oxidase | + |
| Catalase | + |
| Wachstum | |
| anaerob | - |
| 37/41°C | +/- |

| | |
|---|---|
| pH 5.6 | + |
| Mac-Conkey-Agar | - |
| Cetrimid-Agar | - |
| Pigmente | gelb |
| nicht diffundierend | + |
| diffundierend | - |
| fluoreszierend | - |
| Pyocyanin | - |
| Säure aus | |
| Glucose aerob | + |
| Glucose anaerob | - |
| Gas aus Glucose | - |
| Säure aus | |
| Fructose | + |
| Xylose | + |
| Lactose | + |
| Maltose | + |
| Trehalose | + |
| Arabinose | + |
| Cellobiose | + |
| Rhamnose | + |
| Ethanol | - |
| Glycerin | - |
| Dulcit | - |
| Sorbit | - |
| Adonit | - |
| β-Galactosidase | + |
| Alkoholdehydrogenase | - |
| Lysindecarboxylase | - |
| Ornithindecarboxylase | - |
| Acetoinproduktion: | |
| (Voges-Proskauer-Reaktion) | - |
| Indol | - |
| $NO_2^-$ aus $NO_3^-$ | - |
| Denitrifikation | - |
| Phenylalanindeaminase | - |
| Levan aus Saccharose | - |

3

| | |
|---|---|
| Lecithinase | - |
| Urease | - |
| Hydrolyse von | |
| Stärke | + |
| Gelatine | - |
| Casein | - |
| DNA | + |
| Tween 80 | + |
| Äsculin | + |
| Tyrosin-Abbau | - |
| Wuchsstoffbedarf | - |
| Substratverwertung | |
| Acetat | + |
| Adipat | - |
| Caprat | - |
| Citrat | - |
| Glycolat | - |
| Lävulinat | - |
| Malat | + |
| Malonat | - |
| Phenylacetat | - |
| L-Arabinose | + |
| Fructose | + |
| Glucose | + |
| Mannose | + |
| Maltose | + |
| Xylose | + |
| Mannitol | - |
| Gluconat | - |
| 2-Ketogluconat | - |
| N-Acetylglucosamin | + |
| L-Serin | - |

Der Stamm zeigt das für Pseudomonaden typische Fettsäuremuster ohne Cyclopropan-fettsäuren.

Anstelle des Stammes DSM 4429 können auch dessen Varianten und Mutanten eingesetzt werden, sofern sie das genannte Polysaccharid synthetisieren.

Solche Mutanten können in an sich bekannter Weise durch physikalische Mittel, beispielsweise Bestrahlung, wie mit Ultraviolett- oder Röntgenstrahlen oder chemische Mutagene, wie beispielsweise Ethylmethansulfonat (EMS), N-Methyl-N'-nitro-N-nitroso-guanidin (MNNG) oder 2-Hydroxy-4-methoxy-benzophenon (MOB) erzeugt werden.

Als bevorzugte Kohlenstoffquellen für die aerobe Fermentation eignen sich assimilierbare Kohlenhydrate und Zuckeralkohole, wie Glucose, Saccharose, Fructose, Lactose oder D-Mannose, sowie kohlenhydrathaltige Produkte, wie Melasse, Molke oder Malzextrakt. Als bevorzugte stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der baumwollpflanze, Destillationsdrückstände der Alkoholherstellung, Fleischmehle oder Hefeextrakte, aber auch Ammoniumsalze und Nitrate. Außerdem kann die Nährlösung beispielsweise Chloride, Carbonate, Sulfate oder Phosphate der Alkali- oder Erdalkalimetalle, Eisen, Zink und Mangan als zusätzliche anorganische Salze enthalten.

Die Bildung der Verbindung des erfindungsgemäßen Polysaccharids verläuft besonders gut in einer Nährlösung, die Glucose, Saccharose und/oder Melasse in Konzentrationen von 20 bis 120 g/l, bevorzugt von 30 bis 70 g/l enthält.

Die Fermentation erfolgt aerob, also beispielsweise submers unter Schütteln oder Rühren in Schüttelkolben oder Fermentern, gegebenenfalls unter Einführen von Luft oder Sauerstoff, wobei die Belüftungsrate 1 bis 2 l pro Fermenter Volumen mit einer Drucküberlagerung von 1,4 bar, vorzugsweise 1,1 bis 1,6 l pro l sein sollte. Die Fermentation kann in einem Temperaturbereich von etwa 20 bis 40°C, vorzugsweise bei etwa 25 bis 30°C erfolgen. Der pH-Wert sollte in einem Bereich von 5 bis 8,5, bevorzugt 6,5 bis 8 gehalten werden. Man kultiviert den Mikroorganismus unter den genannten Bedingungen bis die stationäre Phase erreicht ist, etwa 2 bis 7 Tage, bevorzugt 3 bis 4 Tage. Man erzielt so eine Polymermenge von 30 bis 70 g/l Nährlösung. Das Polymer besitzt einen hohen Anteil an L-Rhamnose, sowie als weitere wesentliche Bestandteile Glucose, Galaktose, Mannose un Glucuronsäure. Diese Bestandteile liegen etwa in dem Verhältnis 1(Rhamnose):0,5 bis 1,8(Glucose):0,04 bis 0,1(Galaktose): 0,04 bis 0,16(Mannose):0,2 bis 0,6-(Glucuronsäure) vor. Insbesondere liegen die Bestandteile in dem Verhältnis 1:0,5 bis 1:0,05 bis 0,07:0,05 bis 0,07:0,3 bis 0,4 vor.

Vorteilhaft kultiviert man in mehreren Stufen, d.h. man stellt zunächst eine oder mehrere Vorkulturen in einem flüssigen Nährmedium her, die kann in das eigentliche Produktionsmedium, die Hauptkultur, beispielsweise im Volumenverhältnis 1:10, überimpft werden. Eine kontinuierliche Nachfütterung nach 40 bis 120 Stunden Wachstum mit 0,1 bis 2 g/l·h Kohlenhydrate, insbesondere Glucose, Saccharose oder Melasse, ist ebenfalls vorteilhaft.

Die Hydrolyse der Polysaccharide erfolgt in bekannter Weise, z.B. mit wäßrigen Säuren wie Salzsäure, Schwefelsäure, Phosphorsäure oder Essigsäure, zweckmäßig bei erhöhter Temperatur zwischen 100 bis 170°, vorzugsweise bei etwa 80 bis 120°C.

Die Hydrolyse ist im allgemeinen nach 30 bis 180 Minuten, vorzugsweise nach 60 Minuten abgeschlossen. Je nach Art der eingesetzten Säure kann diese durch Destillation oder neutralisierende Fällung entfernt und die säurefreie zuckerhaltige Lösung in bekannter Weise aufgetrennt werden.

Die Abtrennung des gewünschten Desoxyzuckers erfolgt durch chromatographische Methoden, beispielsweise durch Ionenaustauschchromatographie, oder Adsorption an geeigneten oberflächenreichen Substanzen wie Zeolithen.

In den folgenden Beispielen wird die Erfindung weitergehene erläutert. Prozentagaben beziehen sich auf das Gewicht, sofern nicht anderes angegeben ist.

**Beispiel 1**

Die Kultivierung von Pseudomonas paucimobilis DSM 4429 erfolgt in einer Nährlösung mit folgender Zusammensetzung:

| Glucose | 45 g/l |
|---|---|
| Cornsteep | 5 g/l |
| $NaNO_3$ | 2 g/l |
| $K_2HPO_4$ | 1 g/l |
| $MgSO_4 \cdot 7H_2O$ | 1,5 g/l |
| Spurenelementlösung | 2,5 ml/l |
| pH vor Sterilisation | 7.8 |
| Spurenelementlösung: | 3 g/l $CaCl_2 \cdot 2H_2O$ |
| | 1 g/l Fe-III-citrat |
| | 0,2 g/l $MnSO_4$ |
| | 0,1 g/l $ZnCl_2$ |
| | 0,025 g/l $CuSO_4 \cdot 5H_2O$ |
| | 0,02 g/l $Na_2B_4O_7 \cdot 10H_2O$ Natriumtetraborat |
| | 0,004 g/l $CoCl_2$ |
| | 0,01 g/l $Na_2MoO_4 \cdot 2H_2O$ Natriummolybdat |

Die Kultur wurde 7 Tage bei 30°C unter Schütteln inkubiert. Das Polymer wurde durch Zugabe von doppelten Volumina Isopropanol aus der Nährlösung gefällt. 6 g des getrockneten Polymers enthielten 1,98 g L-Rhamnose, 2,6 g Glucose, 0,9 g Glucuronsäure, 0,3 g Mannose und 0,2 g Galaktose.

## Beispiel 2

Pseudomonas paucimobilis DSM 4429 wurde in folgender Nährlösung kultiviert:

| D-Glucose | 45 g/l |
|---|---|
| D-Mannose | 5 g/l |
| $NaNO_3$ | 6 g/l |
| $K_2HPO_4$ | 1 g/l |
| $MgCl_2 \cdot 6H_2O$ | 1,2 g/l |
| $NaS_2O_3 \cdot 5H_2O$ | 1,5 g/l |
| Spurenelementlösung (nach Beispiel 1) | 2,5 ml/l |
| pH | 7.8 |

In 15 g des getrockneten Polymers waren 4,8 g L-Rhamnose, 6 g Glucose, 3,0 g Glucuronsäure, 0,56 g Galaktose und 0,26 g Mannose enthalten.

## Beispiel 3

Ein 14 l Fermenter wurde mit 10 l einer Nährlösung nach Beispiel 2 angesetzt und mit einer 24 Stunden alten Vorkultur aus dem gleichen Medium mit 1 % beimpft.

Die Temperatur der Kultur wurde auf 30°C und der pH-Wert auf 7,0 konstant gehalten und unter Rühren mit 1,5 l VVm Luft begast. Nach 4 Tagen wird das gebildete Polymer durch saure Hydrolyse aufgeschlossen und der Zuckergehalt durch quantitative HPLC betimmt. In einem Liter Kulturbrühe befanden sich 2,5 g L-Rhamnose, 0,2 g Galaktose, 2 g Glucose, 1,4 g Glucuronsäure und 0,3 g Mannose.

## Beispiel 4

Der Stamm wurde wie in Beispiel 3 kultiviert. Nach 40 Stunden wurde mit einer kontinuierlichen Glucosefütterung begonnen. Sie erfolgte mit einer Rate von 0,6 g/l·h aus einem Vorratsgefäß mit einer Glucosekonzentration von 200 g/l. Nach einer Fermentationszeit von insgesamt 70 Stunden wurden die Zuckermengen im Polymer bestimmt. Sie betrugen 4,5 g/l L-Rhamnose, 4 g/l Glucose, 2,8 g/l Glucuronsäure, 0,2 g/l Mannose und 0,2 g/l Galaktose.

**Beispiel 5**

Weitere Charakterisierung des Polymers

Viskositätsmessungen mit dem Viskosimeter Rotovisco RV12 (Fa. Haake Meßtechnik GmbH & Co., 75 Karlsruhe, BRD)

a) bei 30° C

| 1 %ige wäßrige Lösung: | | | | | | |
|---|---|---|---|---|---|---|
| UPM | 0,1 | 0,4 | 1 | 2 | 16 | 64 |
| Viskosität [mPa•s] | 48375 | 28125 | 17475 | 11681 | 3164 | 1058 |
| 0,3 %ige wäßrige Lösung: | | | | | | |
| UPM | 0,1 | 0,4 | 1 | 2 | 16 | 64 |
| Viskosität [mPa•s] | 13125 | 6562 | 4012 | 2381 | 515 | 229 |

b) bei verschiedenen Temperaturen und 2 UPM

| 1 %ige wäßrige Lösung: | |
|---|---|
| Temperatur | Viskosität [mPa•s] |
| 30° C | 11681 |
| 40° C | 10462 |
| 121° C (15 min. Erhitzen, Abkühlen auf 30° C) | 7443 (36 % Viskositätsverlust durch Autoklavieren) |

**Ansprüche**

1. Polysaccharid mit den wesentlichen Bestandteilen L-Rhamnose, Glucose, Galaktose, Mannose und Glucuronsäure in dem Verhältnis 1(Rhamnose):0,5 bis 1,8(Glucose):0,04 bis 0,1(Galaktose):0,04 bis 0,16-(Mannose):0,2 bis 0,6 (Glucuronsäure).

2. Polysaccharid nach Anspruch 1 mit den wesentlichen Bestandteilen in dem Verhältnis 1:0,5 bis 1:0,05 bis 0,07: 0,05 bis 0,07:0,3 bis 0,4.

3. Verfahren zur Herstellung des Polysaccharids nach Anspruch 1 durch Kultivierung eines Pseudomonasstamms, dadurch gekennzeichnet, daß Pseudomonas paucimobilis DSM 4429 oder dessen Varianten oder Mutanten kultiviert werden.

4. Verfahren nach Anspruch 3, dadurch gekennzeichnet, daß der Mikroorganismus in einer Nährlösung, die Glucose, Saccharose und/oder Melasse in Konzentrationen von 2 bis 12% enthält, bezogen auf das gesamte Gewicht des Nährmediums, kultiviert wird.

5. Verfahren nach Anspruch 3 oder 4, dadurch gekennzeichnet, daß bei einem pH-Wert von 5 bis 8,5 kultiviert wird.

6. Verfahren nach einem oder mehreren der Ansprüche 3 bis 5, dadurch gekennzeichnet, daß bei einer Temperatur von 20 bis 40° C kultiviert wird.

7. Pseudomonas paucimobilis DSM 4429 sowie dessen Mutanten und Varianten sofern sie das Polysaccharid nach Anspruch 1 synthetisieren.

8. Verwendung des Polysaccharids nach Anspruch 1 zur Herstellung von Rhamnose.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 102 535 (HOECHST)<br>* Zusammenfassung *<br>--- | 8 | C 08 B 37/00<br>C 12 P 19/04 //<br>(C 12 P 19/04<br>C 12 R 1:38 ) |
| A | JOURNAL OF APPLIED BACTERIOLOGY, Band 62, 1987, Seiten 147-150, Oxford, GB; A. ANSON et al.: "A bacterium yielding a polysaccharide with unusual properties"<br>* Zusammenfassung *<br>--- | 1,3,7 | |
| A | GB-A-2 058 106 (MERCK)<br>* Zusammenfassung *<br>----- | | |

| | RECHERCHIERTE SACHGEBIETE (Int. Cl.4) |
|---|---|
| | C 12 P<br>C 08 B<br>C 13 K |

Der vorliegende Recherchenbericht wurde für alle Patentansprüche erstellt

| Recherchenort | Abschlußdatum der Recherche | Prüfer |
|---|---|---|
| DEN HAAG | 25-07-1989 | SOMERVILLE F.M. |